# EUROPÄISCHE PATENTSCHRIFT

(11) **EP 0 623 579 B1**
(45) Veröffentlichungstag und Bekanntmachung des Hinweises auf die Patenterteilung: **18.09.1996**
(21) Anmeldenummer: 94106218.4
(22) Anmeldetag: 21.04.1994
(51) Int. Cl.: C07C 59/125, C07C 51/367, C07C 67/31

(54) **Verfahren zur Herstellung von Ethercarbonsäuren**
Process for preparation of ether carboxylic acids
Procédé de préparation d'acides éther-carboxyliques

(30) Priorität: 04.05.1993 DE 4314627
(43) Veröffentlichungstag der Anmeldung: 09.11.1994
(73) Patentinhaber: BAYER AG, 51368 Leverkusen (DE)
(72) Erfinder: Sanders, Dr. Josef, D-51373 Leverkusen (DE); König, Dr. Klaus, D-51519 Odenthal (DE)

(56) Entgegenhaltungen:
- Keine einschlägigen Dokumente gefunden

## Beschreibung

Die Erfindung betrifft ein neues Verfahren zur Herstellung von Ethercarbonsäuren durch Anlagerung von Alkoholen an tert.-Alkylester von α,β-ungesättigten Carbonsäuren und anschließende saure Hydrolyse der entstandenen β-Ethercarbonsäureester.

Die DD-A-212 733 beschreibt ein Verfahren zur Herstellung von 3-Alkoxy-propionaten durch Umsetzung von Methylacrylat mit niederen Monoalkoholen in Gegenwart von Ni-Acetoacetonat und Butyllithium. Die EP-A 254 291 beschreibt ein Verfahren zur Herstellung von gegebenenfalls substituierten 3-Alkoxypropionaten durch Umsetzung von Mono- oder Diolen mit gegebenenfalls substituierten Acrylestern in Gegenwart von in der Basenform vorliegenden anionischen Ionenaustauschern. Die EP-A-291 207 beschreibt 3-Alkoxypropionsäuren, hergestellt durch Umsetzung von aliphatischen Diolen bzw. von Alkoxylierungsprodukten aliphatischer Diole mit Acrylnitril in DMF und anschließende Hydrolyse der erhaltenen 3-Alkoxy-propionitrile.

Bei den Verfahrensprodukten der genannten Vorveröffentlichungen handelt es sich zwar um Zwischenprodukte zur Herstellung von β-Ethercarbonsäuren, jedoch sind alle diese Verfahren mit Nachteilen behaltet, die ihren kommerziellen Einsatz erschweren. So ist das in DD-A-212 733 empfohlene Katalysatorsystem relativ teuer und außerdem nur schwer und mit großem Aufwand aus den entsprechenden Additionsverbindungen zu entfernen. Das Verfahren ist im übrigen auf Monoalkohole als Ausgangsmaterial beschränkt. Das Verfahren gemäß EP-A-254 291 ist auf Mono- und Diole beschrankt; außerdem ist zur Erzielung eines akzeptablen Umsetzungsgrades ein hoher Überschuß der Alkoholkomponente bezogen auf die α,β-ungesättigten Carbonsäureester erforderlich. Ebenfalls auf die Verwendung von Diolen beschränkt ist das Verfahren der EP-A-291 207, bei welchem außerdem nachteilig ist, daß hier Dimethylformamid als Lösungsmittel erforderlich ist, was aus dem Additionsprodukt nur sehr schwer entfernt werden kann. Auch die Hydrolyse des erhaltenen Zwischenprodukts ist problematisch. Verwendet man, wie in der EP-A-291 207 beschrieben, konzentrierte Salzsäure im Überschuß, so muß während der Hydrolyse mit unerwünschten Etherspaltungsreaktionen gerechnet werden. Falls jedoch die Hydrolyse in Gegenwart von starken Basen wie Natrium- oder Kaliumhydroxid durchgeführt wird, werden mindestens äquivalente Mengen an Basen benötigt, wodurch später die Freisetzung der Säuren durch Neutralisation, beispielsweise mit Salzsäure, entsprechende hohe Mengen an Salz ergibt. Im übrigen ist allen angeführten Verfahren des Standes der Technik, die von Methyl- oder Ethyl-(meth)acrylaten ausgehen, gemeinsam, daß unerwünschte Nebenreaktionen durch Umesterung des eingesetzten Acrylats unter Freisetzung von Methanol bzw. Ethanol ablaufen, welche wiederum an das eingesetzte Acrylat addieren können, was selbstverständlich auf eine weitere unerwünschte Nebenreaktion hinausläuft.

Jetzt wurde überraschend gefunden, daß man β-Ethercarbonsäuren ohne die oben angesprochenen Nachteile herstellen kann, wenn man ein- oder mehrwertige Alkohole der nachstehend näher genannten Art in Gegenwart von Katalysatoren der nachstehend beispielhaft genannten Art an tert.-Alkylester von α,β-ungesättigten Carbonsäuren der nachstehend beispielhaft genannten Art addiert und anschließend die so erhaltenen Ester in Gegenwart verdünnter Mineralsäuren hydrolysiert. Durch die Verwendung von tert.-Alkylestern der α,β-ungesättigten Carbonsäuren werden Nebenreaktionen der genannten Art vermindert und dadurch Ausbeute und Qualität der Endprodukte verbessert. Im übrigen bereitet die Entfernung des tert.-Alkylrests im Verlauf der Hydrolysereaktion keinerlei Schwierigkeiten, so daß auch hier Nebenreaktionen wie z.B. eine Etherspaltung vermieden werden können,

Gegenstand der Erfindung ist ein Verfahren zur Herstellung von Ethergruppen aufweisenden Carbonsäuren der Formel 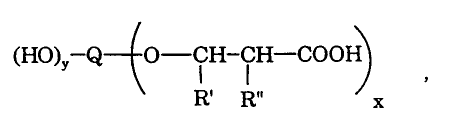 dadurch gekenzeichnet, daß man in einem ersten Verfahrensschritt 1 Mol eines Alkohols der Formel

Q(OH)_{x+y}

in Gegenwart von die Addition von alkoholischen Hydroxylgruppen an α,β-ungesättigte Carbonsäurederivate beschleunigenden Katalysatoren bei 0 bis 100° C mit mindestens x Mol an tert.-Alkylestern von α,β-ungesättigten Carbonsäuren der Formel 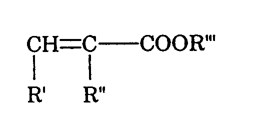 im Sinne einer Additionsreaktion umsetzt und anschließend in einem zweiten Reaktionsschritt die so erhaltenen tert.-Alkylester der entsprechenden β-Ethercarbonsäuren in Gegenwart von Säuren hydrolysiert, wobei in den genannten Formeln
- Q: für einen Rest steht, wie er durch Entfernung der Hydroxylgruppen aus einem (x + y)-wertigen Alkohol des Molekulargewichtsbereichs 32 bis 6000 erhalten wird,
- R' und R'': für gleiche oder verschiedene Reste stehen und Wasserstoff oder eine Methylgruppe bedeuten, wobei mindestens einer der Feste Wasserstoff bedeutet,
- R''': für einen tert.-Alkylrest steht,
- x: für eine Zahl von 1 bis 6, und
- y: für eine Zahl von 0 bis 5 stehen, wobei die Summe x + y einen Wert von 1 bis 6 ergibt.

Ausgangsverbindungen für das erfindungsgemäße Verfahren sind Alkohole der Formel

Q(OH)_{x+y}.

In dieser Formel haben Q, x und y die bereits obengenannte Bedeutung.

Vorzugsweise steht Q für einen Ethergruppen aufweisenden Fest, wie er durch Entfernung der Hydroxylgruppen aus einem Polyetherdiol und -triol des Molekulargewichtsbereichs 300 bis 6000 erhalten werden kann.

x steht vorzugsweise für eine Zahl von 1 bis 3 und y für eine ZahI von 0 bis 2, wobei die Summe x + y vorzugsweise 2 oder 3 ergibt. Gemische von Polyetherdiolen und -triolen der genannten Definition können selbstverständlich ebenfalls als Ausgangsmaterial eingesetzt werden.

Konkrete Beispiele für geeignete Alkohole der Formel

Q(OH)_{x+y}

sind Methanol, Ethanol, die isomeren Propanole, Butanole, Pentanole, Hexanole, höhere Fettalkohole wie z.B. Stearylalkohol, Ethylenglykol, die isomeren Propylenglykole, Butanole, Pentanole, Hexanole, Dihydroxycyclohexane, Di(hydroxymethyl)cyclohexane, Glycerin, Trimethylolpropan, Sorbit, Saccharose, Pentaerythrit und Dipentaerythrit. Geeignet sind insbesondere Polyetherpolyole oder Gemische von Polyetherpolyolen wie sie z.B. durch Anlagerung von Propylenoxid und/oder Ethylenoxid an die oben aufgefürten Alkohole erhalten werden, wobei die Funktionalität der Startermoleküle und der Alkoxylierungsgrad den gemachten Angaben bezüglich des Restes Q und der Indices x und y entsprechen. Besonders bevorzugt werden solche Polyetherpolyole der genannten Formel eingesetzt, die ein Molekulargewicht von 300 bis 4000 und eine (mittlere) Hydroxylfunktionalität von 2 bis 3 aufweisen. Es handelt sich um an sich bekannte Polyetherpolyole der Polyurethanchemie wie sie beispielsweise in EP-A-380 993 beschrieben sind.

Bei den tert.-Alkylestern der Formel 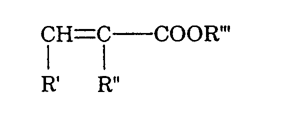 handelt es sich vorzugsweise um tert.-Butylester der Acrylsäure, Methacrylsäure, Crotensäure oder Isocrotonsäure. tert.-Butylacrylat und tert.-Butylmethacrylat sind besonders bevorzugt.

Geeignete Katalysatoren für die Anlagerungsreaktion der Alkohole an die α,β-ungesättigten Carbonsäureester sind beispielsweise Alkalimetallorganyle wie z.B. Butyllithium oder Phenyllithium, Grignardverbindungen wie z.B. Ethylmagnesiumbromid, Ammonium-, Alkali- und Erdalkalihydroxide, Alkali- und Erdalkali-Alkoholate und -Phenolate, Amine wie z.B. Triethylamin oder Pyridin, Guanidine, Phosphine, in der OH-Form vorliegende Ionenaustauscher, Ni-Acetylacetonat, Dialkylzinuoxide. Besonders bevorzugt sind Natriurimhydroxid, Kaliumhydroxid und Kalium-tert.-butanolat.

Die Umsetzung der Ausgangsmaterialien in Gegenwart der Katalysatoren erfolgt in an sich bekannter Weise in einem Lösungsmittel oder bevorzugt in Substanz innerhalb des Temperaturbereiches 0 bis 100°C, vorzugsweise 20 bis 80°C, bis die theoretisch zu erwartende Hydroxylzahl des Umsetzungsproduktes erreicht ist, bzw. bis die Hydroxylzahl sich nicht mehr ändert. Pro Mol Alkohol der Formel

Q(OH)_{x+y}

werden bei der Durchführung der ersten Stufe des erfindungsgemäßen Verfahrens mindestens x Mol, vorzugsweise mindestens 1,1 x Mol des tert.-Alkylesters der ungesättigten Carbonsäure eingesetzt, wobei x die obengenannte Bedeutung bzw. bevorzugte Bedeutung hat. Da die Umsetzung des tert.-Alkylesters im allgemeinen nicht quantitativ im Sinne des erfindungsgemäßen Verfahrens abläuft, empfiehlt sich auch bei der Herstellung von Hydroxylgruppen aufweisenden Verfahrensprodukten die Verwendung einer überstöchiometrischen Menge (mindestens 1,1 x Mol pro Mol) des x Alkylesters bezogen auf den Alkohol. Setzt man die ungesättigten Carbonsäureester, bezogen auf die Menge der in der Alkoholkomponente enthaltenen Hydroxylgruppen im Unterschuß ein, werden gezielt Produkte erhalten, die noch freie Hydroxylgruppen aufweisen, die für weitere Umsetzungen zur Verfügung stehen. Andererseits ist zum vollständigen Umsatz der Hydroxylgruppen der Alkohole ein Überschuß der Carbonsäureester vorteilhaft bzw. erforderlich. Dieser Überschuß an Carbonsäureester kann nach erfolgter Umsetzung z.B. durch Destillation aus dem Reaktionsgemisch erfernt und anschließend erneut eingesetzt werden.

Die Katalysatoren kommen im allgemeinen in einer Menge von 0,05 bis 20 Mol.-%, vorzugsweise 1 bis 10 Mol.-%, bezogen auf die Gesamtmenge der eingesetzten Hydroxyl-Äquivalente der Alkoholkomponenten, zum Einsatz.

Geeignete Lösungsmittel für diese Umsetzung sind solche, die sich unter den Reaktionsbedingungen inert verhalten. In Frage kommen beispielsweise Diethylether, Ethylenglykoldimethylether, Tetrahydrofüran, Dioxan, tert.-Butanol, Benzol, Toluol, Chlorbenzol, Dimethylformamid, Dimethylsulfoxid, N-Methylpyrrolidon und Acetonitril. Bevorzugt wird die Umsetzung in Substanz ohne Lösungsmittel durchgeführt.

Die Hydrolyse der als Zwischenstufe erhaltenen gegebenenfalls substituierten 3-Alkoxy-proponsäurederivate erfolgt nach an sich bekannten Verfahren beispielsweise durch Umsetzung mit verdünnten, wäßrigen Säuren wie beispielsweise Halogenwaserstoffsäuren, Schwefelsäure, Phosphorsäure, Sulfonsäuren und Halogencarbonsäuren. Bevorzugt ist verdünnte Salzsäure einer Konzentration von 1 bis 10 Gew.-%. Beispielsweise werden die Reaktionskomponenten bei einer Temperatur von 20 bis 100°C, vorzugsweise 60 bis 100°C solange gerührt, bis die Säurezahl einer eingeengten Probe konstant ist. In manchen Fällen ist es vorteilhaft, die Hydrolyse in Gegenwart eines Lösungsmittels durchzuführen, das nach beendeter Reaktion wieder destillativ entfernt und erneut eingesetzt werden kann. In Frage kommen solche Lösungsmittel der oben beispielhaft genannten Art, die unter den Reaktionsbedingungen inert sind.

Die nach dem erfindungsgemäßen Verfahren hergestellten Ethercarbonsäuren zeichnen sich durch niedrige Viskositäten und geringe Eigenfarbe aus. Sie sind z.B. zur Herstellung von Polyetherestern, Polyetheramiden, Polyethercarbonaten und dergleichen geeignet. Sie können auch für sich oder in Form ihrer Salze als Emulgatoren oder als Katalysatoren Anwendung finden. Besonders bevorzugt werden die erfindungsgemäßen Ethercarbonsäuren in Form ihrer Natrium- oder Alkalisalze als einbaufähige Katalysatoren bei der Herstellung von Polyurethanschaumstoffen verwendet.

In den nachfolgenden Beispielen beziehen sich alle Prozentangaben auf das Gewicht.

### Beispiel 1

### a) Acrylataddition

In 2433 g (23,85 OH-Äquivalente) Polyethertriol der OH-Zahl 550, hergestellt durch Propoxylierung von Trimethylolpropan, löst bzw. dispergiert man unter guten Rühren und Erwärmen auf 40°C 28,7 g (0,51 Mol) gepulvertes Kaliumhydroxid. Anschließend tropft man bei dieser Temperatur 1067 g (8,34 Mol) tert.-Butylacrylat gleichmäßig über 5 Stunden zu und rührt anschließend weitere 16 Stunden bei 40°C nach. Nach Zutropfen von 50,5 g (0,51 Mol) 37 %iger Salzsäure werden flüchtige Bestandteile (73 g, in der Hauptsache nicht umgesetztes Acrylat und etwas tert.-Butanol) im Wasserstrahlvakuum bis zu einer Sumpftemperatur von 80°C abdestilliert. Nach Filtration erhält man ein klares, leicht gelbliches Produkt mit einer Säurezahl von 13,9 und einer OH-Zahl von 267,6 mg KOH. Dies entspricht einem Umsatz von 31,9 % der anfangs vorhandenen Hydroxylgruppen. Daraus errechnet sich ein Ester-Äquivalentgewicht von 447,9.

### b) Hydrolyse

Eine Mischung aus 3400 g (7,59 COOR-Äquivalente) Produkt aus Beispiel 1a), 3400 ml Wasser und 149,8 g (1,52 Mol) 37 %ige Salzsäure wird bei 95°C solange gerührt, bis die Destillation von tert.-Butanol zum Erliegen kommt (ca. 8 h). Man stellt den Rührer ab, läßt ca. 30 Minuten absitzen und trennt die (obere) verdünnte Salzsäurephase von der (unteren) Produktphase ab. Anschließend wird verbliebenes Restwasser aus dem Rohprodukt durch Destillation im Vakuum bei 0,5 mbar bis zu einer Sumpftemperatur von 60°C entfernt. Man erhält ein klares, gelbliches Produkt mit einer Säurezahl von 104.

### Vergleichsbeispiel zu Beispiel 1a)

### Acrylataddition

1149 g (11,26 OH-Äquivalent) Polyethertriol gemäß Beispiel 1a), 339 g (3,94 Mol) Methylacrylat, 12,5 g (0,22 Mol) gepulvertes Kaliumhydroxid und 22 g (0,22 Mol) 37 %ige Salzsäure werden analog Beispiel 1a) umgesetzt. Destillat: 158,6 g (hauptsächlich etwa gleich große Mengen Acrylat und Methanol). Man erhält ein trübes, gelbliches Produkt mit einer Säurezahl von 9 und einer OH-Zahl von 329,4. Dies entspricht einem Umsatz von 27,7 % der anfangs vorhandenen Hydroxylgruppen. Daraus errechnet sich ein Ester-Äquivalentgewicht von 468,7.

### Beispiel 2

### a) Acrylataddition

1795 g (17,60 OH-Äquivalent) Polyethertriol gemäß Beispiel 1a), 1705 g (13,32 Mol) tert.-Butylacrylat, 20,9 g (0,37 Mol) gepulvertes Kaliumhydroxid und 36,8 g (0,37 Mol) 37 %ige Salzsäure werden analog Beispiel 1a) umgesetzt. Destillat: 175 g (in der Hauptsache Acrylat und etwas tert.-Butanol). Man erhält ein Klares, leicht gelbliches Produkt mit einer Säurezahl von 10,7 und einer OH-Zahl von 114,1. Dies entspricht einem Umsatz von 62,9 % der anfangs vorhandenen Hydroxylgruppen. Daraus errechnet sich ein Ester-Äquivalentgewicht von 290,2.

### b) Hydrolyse

1555 g (5,36 COOR-Äquivalent) Produkt aus Beispiel 2a), 1555 g Wasser und 105,7 g (1,07 Mol) 37 %ige Salzsäure werden analog Beispiel 1b) umgesetzt. Man erhält ein klares, gelbliches Produkt mit einer Säurezahl von 184,9.

### Vergleichsbeispiel zu Beispiel 2a)

### Acrylataddition

913 g (8,95 OH-Äquivalent) Polyethertriol gemäß Beispiel 1a), 577 g (6,71 Mol) Methylacrylat, 10 g (0,18 Mol) gepulvertes Kaliumhydroxid und 17,6 g (0,18 MJol) 37 %ige Salzsäure werden analog Beispiel 2a) umgesetzt. Destillat: 241 g (hauptsächlich Methylacrylat neben Methanol). Man erhält ein trübes, gelbliches Produkt mit einer Säurezahl von 10,1 und einer OH-Zahl von 330,4. Dies entspricht einem Umsatz von 26,5 % der anfangs vorhandenen Hydroxylgruppen. Daraus errechnet sich ein Ester-Äquivalentgewicht von 470,9.

In den nicht erfindungsgemäßen Vergleichsbeispielen zu den Beispielen 1a) und 2a) wurden anstelle von tert.-Butylacrylat jeweils äquimolare Mengen Methylacrylat eingesetzt. Die erhaltenen Umsätze sind deutlich niedriger als die mit tert.-Butylacrylat erzielten Umsätze. Im Fall von Methylacrylat enthalten die Destillate beträchtliche Anteile an Methanol, was auf Verseifung des Methylacrylats durch den basischen Katalysator hindeutet. Dies wird durch die Umsatzrate des Vergleichsbeispiels 2 bestätigt, die trotz deutlich höherem Methylacrylateinsatz nicht höher als die Umsatzrate des Vergleichsbeispiels 1 ist. Offenbar wurde hier der Katalysator verbraucht, wodurch die Reaktion schon nach geringem Umsatz zum Erliegen kam.

### Beispiel 3

### a) Acrylataddition

295 g (1 OH-Äquivalent) Polyetherdiol der OH-Zahl 190, hergestellt durch Ethoxylierung von Propylenglykol, 128 g (1 Mol) tert.-Butylacrylat, 0,6 g (0,01 Mol) gepulvertes Kaliumhydroxid und 1,1 g (0,01 Mol) 37 %ige Salzsäure werden analog Beispiel 1a) umgesetzt. Destillat: 24 g (hauptsächlich Acrylat und etwas tert.-Butanol). Man erhält ein Klares, schwach gelbliches Produkt der OH-Zahl 21,2. Dies entspricht einem Umsatz von 84,8 % der anfangs vorhandenen Hydroxylgruppen. Daraus errechnet sich ein Ester-Äquivalentgewicht von 476,1.

### b) Hydrolyse

160 g (0,34 COOR-Äquivalent) Produkt aus Beispiel 3a), 45 g Wasser und 16,6 g (0,16 Mol) 37 %ige Salzsäure werden analog Beispiel 1b) umgesetzt. Man erhält ein Klares, fast farbloses Produkt mit einer Säurezahl von 142.

### Beispiel 4

### a) Acrylataddition

1043 g (4,66 OH-Äquivalent) Polyethertriol der OH-Zahl 250, hergestellt durch Propoxylierung von Trimethylolpropan und anschließende Ethoxylierung des Propoxylierungsprodukts (PO:EO-Gewichtsverhältnis = 1,1:98,9), 452 g (3,53 Mol) tert.-Butylacrylat, 5,2 g (0,09 Mol) gepulvertes Kaliumhydroxid und 9,2 g (0,09 Mol) 37 %ige Salzsäure werden analog Beispiel 1a) umgesetzt. Destillat: 97 g (hauptsächlich Acrylat und etwas tert.-Butanol). Man erhält ein klares, schwach gelbliches Produkt der OH-Zahl 57,7. Dies entspricht einem Umsatz von 66 % der anfangs vorhandenen Hydroxylgruppen. Daraus errechnet sich ein Ester-Äquivalentgewicht von 467.6.

### b) Hydrolyse

150 g (0,32 COOR-Äquivalent) Produkt aus Beispiel 4a), 136,5 g Wasser und 13,5 g (0,14 Mol) 37 %ige Salzsäure werden analog Beispiel 1b) umgesetzt. Man erhält ein Klares, schwach gelbliches Produkt mit einer Säurezahl von 114.

## Patentansprüche

1. Verfahren zur Herstellung von Ethergruppen aufweisenden Carbonsäuren der Formel 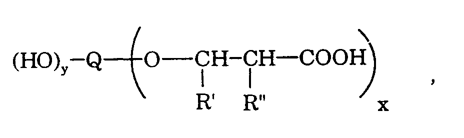 dadurch gekenzeichnet, daß man in einem ersten Verfahrensschritt 1 Mol eines Alkohols der Formel
Q(OH)_{x+y}
in Gegenwart von die Addition von alkoholischen Hydroxylgruppen an α,β-ungesättigte Carbonsäurederivate beschleunigenden Katalysatoren bei 0 bis 100°C mit mindestens x Mol an tert.-Alkylestern von α,β-ungesättigten Carbonsäuren der Formel 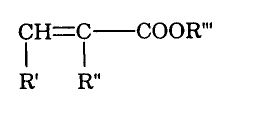 im Sinne einer Additionsreaktion umsetzt und anschließend in einem zweiten Reaktionsschritt die so erhaltenen tert.-Alkylester der entsprechenden β-Ethercarbonsäuren in Gegenwart von Säuren hydrolysiert, wobei in den genannten Formeln
Q für einen Rest steht, wie er durch Entfernung der Hydroxylgruppen aus einem (x + y)-wertigen Alkohol des Molekulargewichtsbereichs 32 bis 6000 erhalten wird,
R' und R'' für gleiche oder verschiedene Reste stehen und Wasserstoff oder eine Methylgruppe bedeuten, wobei mindestens einer der Reste Wasserstoff bedeutet,
R''' für einen tert.-Alkylrest steht,
x für eine Zahl von 1 bis 6, und
y für eine Zahl von 0 bis 5 stehen, wobei die Summe x + y einen Wert von 1 bis 6 ergibt.

2. Verfahren gemäß Anspruch 1, dadurch gekennzeichnet, daß man als tert.-Alkylester einer α,β-ungesättigte Carbonsäure, tert.-Butylacrylat oder tert.-Butylmethacrylat verwendet.

3. Verfahren gemäß Anspruch 1 und 2, dadurch gekennzeichnet, daß man als mehrwertige Alkohole der Formel
Q(OH)_{x+y}
Polyetherdiole oder -triole des Molekulargewichtsbereichs 300 bis 6000 oder Gemische von derartigen Polyetherpolyolen verwendet.

4. Verfahren gemaß Anspruch 1 bis 3, dadurch gekennzeichnet, daß man als Katalysator für die Additionsreaktion zwischen ungesättigten Carbonsäureestern und Alkoholen Natriumhydroxid, Kaliumhydroxid oder Kalium-tert.-butanolat verwendet.

## Claims

1. A process for preparing carboxylic acids which contain ether groups, of the formula 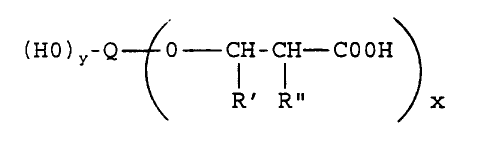 characterised in that, in a first process step, 1 mole of an alcohol of the formula
Q(OH)_{x+y}
is reacted with at least x moles of tert-alkyl esters of α,β-unsaturated carboxylic acids of the formula 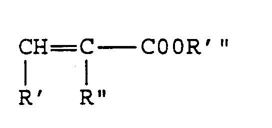 in an addition reaction at 0 to 100°C, in the presence of catalysts which accelerate the addition of alcoholic hydroxyl groups to α,β-unsaturated carboxylic acid derivatives, and then, in a second reaction step, the tert.-alkyl ester obtained in this way is hydrolysed in the presence of acids to give the corresponding β-ethercarboxylic acids, wherein, in the formulas mentioned,
Q represents a radical such as is obtained by removing the hydroxyl groups from a (x+y)-hydric alcohol with a molecular weight in the range 32 to 6000,
R' and R'' represent identical or different radicals and hydrogen or a methyl group, wherein at least one of the radicals is hydrogen,
R''' represents a tert.-alkyl radical,
x represents a number from 1 to 6, and
y represents a number from 0 to 5, wherein the sum x + y has a value from 1 to 6.

2. A process according to Claim 1, characterised in that tert.-butyl acrylate or tert.-butyl methacrylate is used as a tert.-alkyl ester of an α,β-unsaturated carboxylic acid.

3. A process according to Claims 1 and 2, characterised in that polyetherdiols or -triols with a molecular weight in the range 300 to 6000 or mixtures of this type of polyetherpolyol are used as polyhydric alcohols of the formula
Q(OH)_{x+y}.

4. A process according to Claims 1 to 3, characterised in that sodium hydroxide, potassium hydroxide or potassium tert.-butanolate is used as a catalyst for the addition reaction between unsaturated carboxylates and alcohols.

## Revendications

1. Procédé pour la préparation d'acides carboxyliques présentant des groupes éther, répondant à la formule 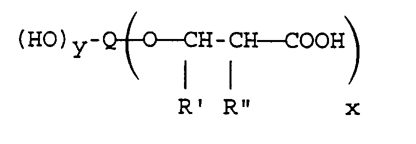 caractérisé en ce que, dans une première étape opératoire, on fait réagir 1 mole d'un alcool de formule
Q(OH)_{x+y}
en présence de catalyseurs accélérant l'addition de groupes hydroxyle alcooliques à des dérivés d'acides carboxyliques à insaturation α,β à une température de 0 à 100°C avec au moins x moles d'esters tert.-alkyliques d'acides carboxyliques à insaturation α,β répondant à la formule: 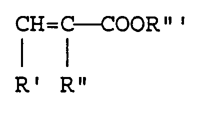 dans le sens d'une réaction d'addition, et ensuite, dans une seconde étape réactionnelle, on hydrolyse en présence d'acides les esters tert.-alkyliques ainsi obtenus des acides β-éther-carboxyliques correspondants, dans lequel, dans les formules indiquées
Q représente un radical tel qu'on l'obtient par élimination des groupes hydroxyle d'un alcool à valence (x+y) du domaine de poids moléculaire de 32 à 6000,
R' et R'' représentent des radicaux identiques ou différents et désignent un atome d'hydrogène ou un groupe méthyle, au moins un des radicaux représentant un atome d'hydrogène,
R''' représente un radical tert.-alkyle,
x représente un nombre de 1 à 6, et
y représente un nombre de 0 à 5, la somme x + y donnant une valeur de 1 à 6.

2. Procédé selon la revendication 1, caractérisé en ce qu'on utilise, comme ester tert.-alkylique d'un acide carboxylique à insaturation α,β, l'acrylate de tert.-butyle ou le méthacrylate de tert.-butyle.

3. Procédé selon les revendications 1 et 2, caractérisé en ce que, comme alcools polyvalents de formule
Q(OH)_{x+y}
on utilise des polyéther-diols ou -triols du domaine de poids moléculaire de 300 à 6000 ou encore des mélanges de polyétherpolyols de ce type.

4. Procédé selon les revendications 1 à 3, caractérisé en ce qu'on utilise, comme catalyseur pour la réaction d'addition entre des esters carboxyliques insaturés et des alcools, l'hydroxyde de sodium, l'hydroxyde de potassium ou le tert.-butanolate de potassium.
